# EUROPEAN PATENT APPLICATION

(11) **EP 2 975 136 A1**
(43) Date of publication of application: **20.01.2016**
(21) Application number: 14176969.5
(22) Date of filing: 14.07.2014
(51) Int. Cl.: C12Q 1/68

(54) **A method for detecting PCR amplification in a sample**

(71) Applicant: Parlanca Limited, Dublin 2 (IE)
(72) Inventor: Higgins, Janika, Dublin, D6 (IE)
(74) Representative: Purdy, Hugh Barry

(57) **Abstract**

A method for detecting PCR amplification of a target DNA molecule in a sample is described and employs a forward PCR primer having a sequence that is complementary to the target double stranded nucleic acid and a tail sequence, a reverse PCR primer having a sequence that is complementary to the target double stranded nucleic acid, and a dual labelled probe containing an oligonucleotide sequence identical to the tail sequence of the forward PCR primer oligonucleotide, and a reporter label and a quencher-label separated by a nuclease susceptible cleavage site. The method comprises the steps of incubating the forward and reverse PCR primers and dual labelled probe together, performing at least two rounds of PCR to generate a double stranded nucleic acid comprising the tail region and a sequence complementary to the tail region, and initiating a further round of PCR in which the oligonucleotide probe binds to the sequence complementary to the tail region, whereby the oligonucleotide probe is displaced and cleaved resulting in a detectable signal from the reporter label.

## Description

### Background to the Invention

The invention relates to a method of detecting PCR amplification in a sample. In particular, the invention relates to a method of genotyping a sample of DNA for the presence of a first or second allele of a gene within the DNA by means of PCR amplification.

There are several genotyping assay systems available, a well known example being KASP chemistry (EP1726664). This detection system is based on two singly labelled (probe) oligonucleotides, which are hybridised together when the target sequence for the reporter oligonucleotide is not present. One of the oligonucleotides is labelled with a reporter dye and the other with a quencher molecule, thus, the fluoresence of the reporter is quenched (undetectable) when the probe is in its hybridised form, commonly known as a probe cassette. In a bi-allelic SNP detection system, two types of these universal probes are needed. The KASP detection system also utilises two allele specific and a single shared common primers which are designed to target the SNP site. These two allele specific primers are "tailed" with either one of two sequences which are complementary to one of the two universal probes. When the allele specific primer is used in the PCR reaction, the probe will hybridise with the "tail" sequence now present in the newly synthesised DNA, releasing reporter dye from the proximity of the quencher molecule resulting in a fluoresence signal. KASP chemistry is known to have technical constraints with "over-cycling" leading to the changing of fluorescence over time due to random amplification, inappropriate binding, etc.. This leads to issues when trying to genotype multiple samples with varying template DNA quality and concentration, as not all samples reach a fluorescence plateau at the same cycle number. This means that each sample for each probe mix must be analysed multiple times to "track" the fluorescence to best estimate which probe is amplified the most for each sample. Over time KASP probes can become randomly annealed to other points along the sequence as the supplies within the reagent mix are depleted, leaving the fluorophores from either cassette to be cleaved or separated from the quencher molecule and fluoresce permanently regardless of whether or not they should have taken part in the PCR reaction. This is why we see a movement of the clusters either towards the heterozygous group or into a scatter gun style pattern that characterises the issues with KASP performance.

It is an object of the invention to overcome at least one of the above-referenced problems.

### Brief Description of the Invention

Broadly, the invention provides a method for detecting the presence of a target DNA molecule in a sample. Similar to the KASP methodology, the methods of the invention employ unlabelled tailed forward primer molecules and a common reverse primer that are used to initiate synthesis of a target DNA molecule. However, instead of employing a probe that consists of labelled first and second oligonucleotide sequences that hybridise to one another in free solution, the method of the invention employs a dual labelled single stranded probe. Surprisingly, the use of dual labelled probes has been shown to reduce the problem of "over-cycling" to an insignificant number of mis-calls (in comparison to KASP data)/change of calls being reported over multiple cycling. Therefore only one read is necessary, leading to dramatically reduced data review and higher throughput of data compared to its KASP equivalent. Hereafter, the method of the invention is referred to as "GASP" (Gorgeous Allele Specific PCR).

Broadly, the invention provides a method for detecting PCR amplification of a target DNA molecule in a sample, which method employs:
- a forward PCR primer having a sequence that is complementary to the target double stranded nucleic acid and a tail sequence;
- a reverse PCR primer having a sequence that is complementary to the target double stranded nucleic acid; and
- a dual labelled probe containing an oligonucleotide sequence identical to the tail sequence of the forward PCR primer oligonucleotide, and a reporter label and a quencher-label separated by a nuclease susceptible cleavage site,
the method comprising the steps of:
- incubating the forward and reverse PCR primers and dual labelled probe together;
- performing at least two rounds of PCR to generate a double stranded nucleic acid comprising the tail region and a sequence complementary to the tail region; and
- initiating a further round of PCR in which the oligonucleotide probe binds to the sequence complementary to the tail region, whereby the oligonucleotide probe is displaced and cleaved resulting in a detectable signal from the reporter label.

The invention also provides a kit suitable for performing a method of detecting PCR amplification of a target double stranded nucleic acid in a sample, the kit comprising
- a forward PCR primer having a sequence that is complementary to the target double stranded nucleic acid and a tail sequence;
- a reverse PCR primer having a sequence that is complementary to the target double stranded nucleic acid; and
- a dual labelled probe containing an oligonucleotide sequence identical to the tail sequence of the forward PCR primer oligonucleotide, and a reporter label and a quencher-label separated by a nuclease susceptible cleavage site.

In another aspect, the invention provides a method of genotyping a sample of DNA for the presence of a first or second allele of a gene within the DNA by means of PCR amplification, which method employs:
- a first forward PCR primer having a first allele-specific sequence and a first tail sequence;
- a second forward PCR primer having a second allele specific sequence and a second tail sequence different to the first tail sequence;
- a common reverse PCR primer;
- a first labelled probe comprising an oligonucleotide sequence identical to the tail sequence of the first forward PCR primer, and a first reporter label and a quencher-label separated by a nuclease susceptible cleavage site; and
- a second labelled probe containing an oligonucleotide sequence identical to the tail sequence of the second forward PCR primer, and a second reporter label and a quencher-label separated by a nuclease susceptible cleavage site, in which the second reporter label emits a signal that is different to that of the first reporter label,
the method comprising the steps of:
- incubating the forward and reverse PCR primers and probes together;
- initiating at least two rounds of PCR to generate a double stranded nucleic acid comprising the first or second tail region and a sequence complementary to the first or second tail region;
- initialling a further round of PCR in which one of the probes binds to the sequence complementary to the first or second tail region, whereby the oligonucleotide probe is displaced and cleaved resulting in a detectable signal from the reporter label; and
- correlating the detectable signal from the reporter label with the presence of the first or second allele of the gene in the sample of DNA.

The invention also provides a kit suitable for genotyping a sample of DNA for the presence of a first or second allele of a gene within the DNA, the kit comprising:
- a first forward PCR primer having a first allele-specific sequence and a first tail sequence;
- a second forward PCR primer having a second allele specific sequence and a second tail sequence different to the first tail sequence;
- a common reverse PCR primer;
   - a first labelled probe comprising an oligonucleotide sequence identical to the tail sequence of the first forward PCR primer, and a first fluorescent label and a quencher-label separated by a nuclease susceptible cleavage site; and
   - a second labelled probe containing an oligonucleotide sequence identical to the tail sequence of the second forward PCR primer, and a second fluorescent label and a quencher-label separated by a nuclease susceptible cleavage site, in which the second fluorescent has excitation and emission properties that are different to those of the first fluorescent label.

In this specification, the term "PCR" should be understood to mean polymerase chain reaction, which is fully described in US Patent No: 4683195.

In this specification, the term "detecting PCR amplification of a target DNA molecule in a sample" should be understood to mean determining whether a target DNA molecule is present in a sample, or quantitatively determining the presence of the target DNA in the sample.

The term "forward PCR primer" should be understood to mean a short single stranded oligonucleotide sequence designed to act as a point of initiation of synthesis along a complementary part of a first strand of a target DNA molecule.

The term "reverse primer" or common reverse primer" should be understood to mean a short single stranded oligonucleotide sequence designed to act as a point of initiation of synthesis along a complementary part of a second strand of a target DNA molecule.

The term "tail sequence" refers to a part of the forward PCR primer, typically at the 5' end of the primer molecule that is not complementary to the target DNA molecule. Typically, the tail sequence is 20-30, preferably 20-24, nucleotides in length.

The term "dual labelled probe" refers to a single stranded oligonucleotide sequence having a reporter label, a quencher label, and probe sequence disposed between the two labels, and in which the reporter label and quencher label are in sufficient proximity such that the quencher prevents the reporter label emitting a detectable signal. The probe sequence generally includes a sequence that is sensitive to a Taq polymerase. Typically, the probe comprises 20-30 nucleotides, preferably 20-24 nucleotides. In one embodiment, the dual labelled probe is selected from the sequences:
FAM-5' GAA GGT GAC CAA GTT CAT GCT 3'- BHQ1
JOE-5' GAA GGT CGG AGT CAA CGG ATT 3' - BHQ1

The term "reporter label" should be understood to mean a molecule that is capable of emitting a detectable signal and is capable of being quenched by the quencher molecule. Examples of reporter molecules include molecules that are detectable by spectroscopic, photochemical, biochemical, immunochemical, or chemical means. Examples of reporter labels that may be employed include enzymes, enzyme substrates, radioactive atoms, fluorescent dyes, chromophores, chemiluminescent labels, or ligands having specific binding partners. Preferably, the reporter label is detectable by spectroscopy, and ideally is a fluorescent dye. Examples of fluorescent dyes that may be employed with the methods of the present invention include FAM, VIC, LIZ, NED, PET. In embodiments of the invention directed to genotyping a sample of DNA to detect the presence of a first or second allele of the DNA, two different dual-labelled probes will be employed, one having a first reporter label capable of emitting a first signal, and the second having a second reporter label capable of emitting a second signal that is different to the first signal. Examples of first and second fluorescent reporter labels include FAM (abs 495nm, em520nm) and JOE (abs 520nm, em 548nm).

The term "quencher label" should be understood to mean a molecular entity that is capable of quenching the signal emitted by the reporter molecule, when in proximity with the reporter molecule. When the reporter label is a fluorescent dye, the quencher is typically selected from BHQ-1, BHQ-2, BHQ-3, TAMRA.

The term "Taq polymerase" should be understood to mean a DNA polymerase that has a DNA synthesis dependent, strand replacement 5'-3' exonuclease activity (Gelfand, PCR Technology: Principles and Applications for DNA Amplification, Erlich, Ed., Stockton Press, N.Y. (1989), Chapter 2).

The term "genotyping a sample of DNA to detect first or second alleles of a gene within the DNA sample" should be understood to mean determining whether a giver allele of the gene is present in the sample of DNA. Thus, the method of the invention can be employed to detect the presence or absence of single nucleotide polymorphisms in a sample, by using the method of the invention to detect an allele comprising the SNP. Generally, with these methods, it is necessary to include in the reaction mixture two forward primers, one specific to a first allele and a second specific to the second allele, and two dual labelled probes, one specific for the first forward primer and the second specific for the second primer. The reporter molecules on the two probes will be different, thus allowing the signal emitted during the PCR process to be assigned to one or the other probe.

### Example of GASP chemistry in practice

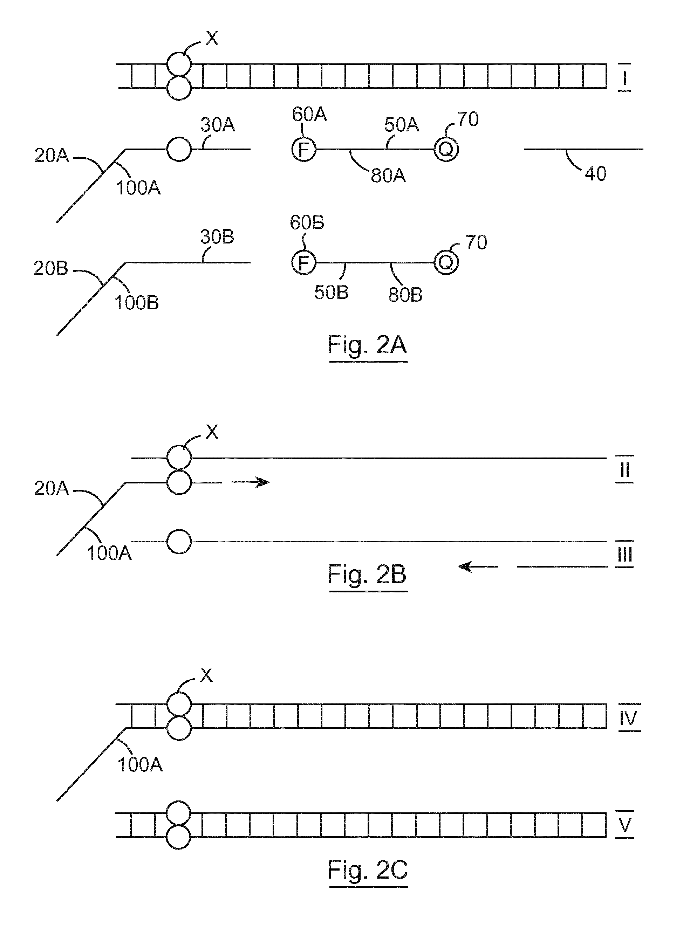

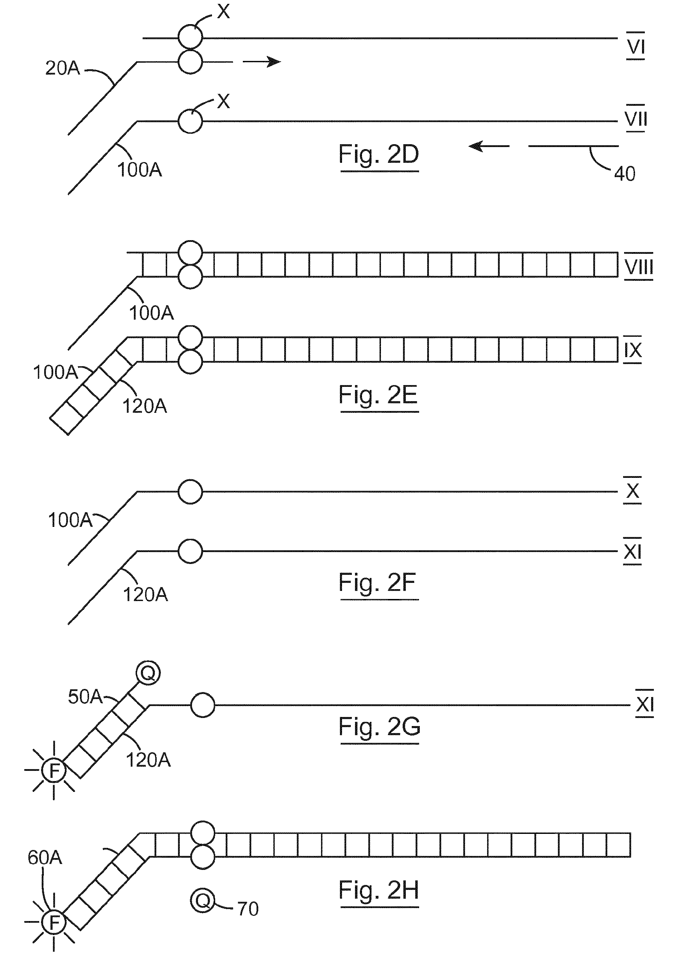

As described previously, and in more detail in figures 2A to 2H, GASP chemistry can be used to identify the presence of known SNPs within a DNA sample. With a panel of SNPs that have been determined through investigation to provide enough unique identifying information so as to be used to both give a unique ID for the sample tested as well as using Mendelian inheritance, used to determine the parents of a sample provided.

In this situation, all known parents are genotyped against the panel of SNPs using the GASP chemistry. Subsequent to this the offspring are also genotyped. Using SNP signature analysis, we can then determine which parents belong to which offspring with high probability. From this data then we can assign parents from within the group to the offspring or we can confirm that the parents of those offspring are not contained within the tested parental group.

The use of GASP in this way has been compared with previous similar work done in house with KASP chemistry (see Fig. 4).

### Brief Description of the Figures

Figs 1A to 1H is an illustration of the steps of a method of detecting PCR amplification of a target nucleic acid according to the invention; and
Figs. 2A to 2H is an illustration of the steps of a method of genotyping a sample of DNA for the presence of a first or second allele of a gene within the DNA by means of PCR amplification.

### Detailed Description of the Invention

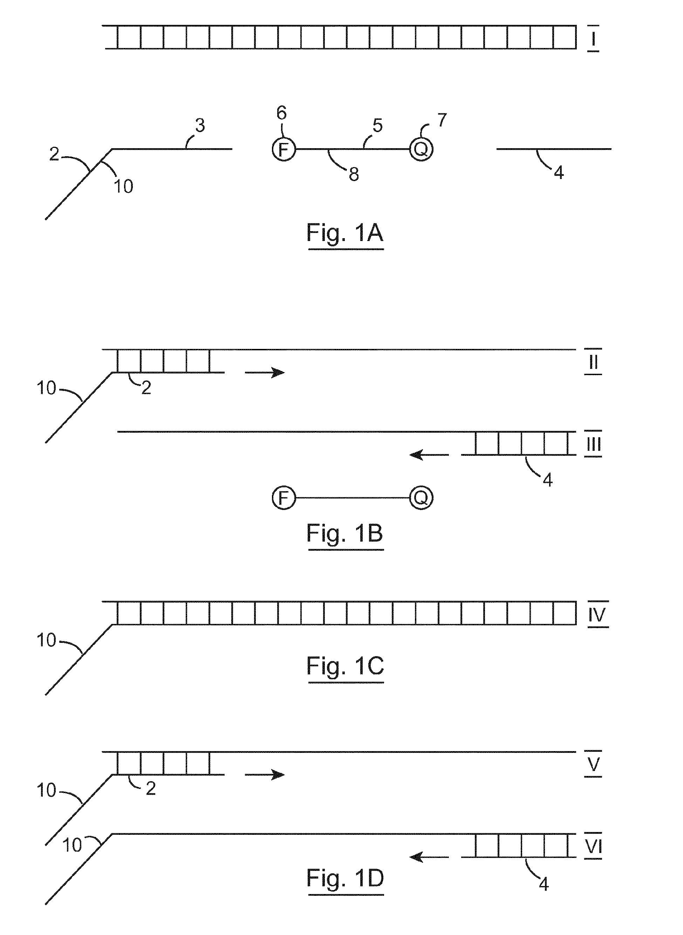

Referring to the Figures, and initially to Figs 1A to 1H, there is illustrated a method of detecting PCR amplification of a target sequence according to the invention.

Referring initially to Fig. 1A, there is illustrated a target DNA molecule I, a forward PCR primer 2 having a first sequence 3 complementary to a sequence at the 5' end of the first strand of the target DNA molecule I, a reverse primer 4 having a sequence complementary to a sequence at the 5' end of the second strand of the target DNA molecule I, and a probe 5 having a fluorescent label 6, a quencher 7, and a probe sequence 8. The forward PCR primer 2 additionally comprises a tail sequence 10 that is identical to the probe sequence 8. The figures do not illustrate the other components required to perform PCR, which include Taq DNA polymerase, deoxy nucleotide triphosphates dNTP's, and reaction buffer, the details of which will be known to a person skilled in the art. Referring to Fig. 1B, a first round of PCR is initiated with a denaturation step and the DNA molecule I denaturing to form single stranded DNA molecules II and III. The forward PCR primer 2 binds to the 5' end of strand II, and the reverse PCR primer 4 binds to the 5' end of the second strand III, in an annealing step. As the probe sequence 8 of the probe 5 is identical with the tail sequence 10 of the primer 2, it will not bind to the tail. Referring to Fig. 1C, there is illustrated a synthesised DNA molecule IV formed as a result of the first round of PCR and elongation of strand II of Fig. 1B, the DNA molecule incorporating the tail sequence 10. Again, as the probe sequence 8 of the probe 5 is identical with the tail sequence 10 of the primer 2, it will not bind to the tail.

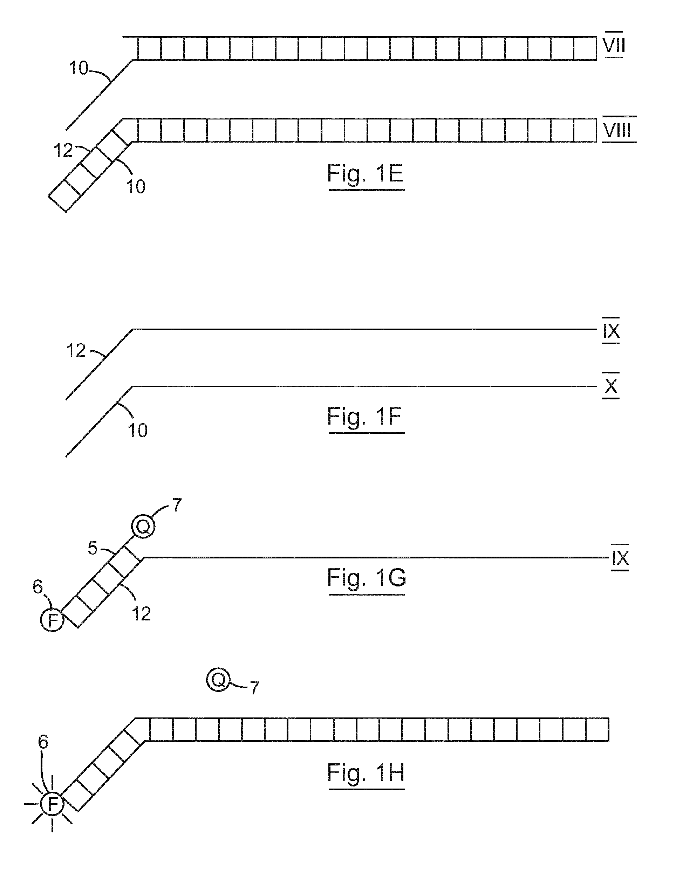

Referring to Fig. 1D, a second round of PCR is initiated with a denaturation step and the DNA molecule IV denaturing to form single stranded DNA molecules V and VI. The forward PCR primer 2 binds to the 5' end of strand V, and the reverse PCR primer 4 binds to the 5' end of the second strand VI, in an annealing step. As the probe sequence 8 of the probe 5 is identical with the tail sequence 10 of the primer 2, it will not bind to the tail. Referring to Fig. 1E, following the elongation step, two new DNA molecules VII and VIII are synthesised, molecule VII comprising the tail sequence 10 in a single stranded forms, and molecule VIII comprising a double stranded tail region, with the tail sequence 10 having a complementary tail region 12.

Referring to Fig. 1F, a third round of PCR is initiated with a denaturation step and the DNA molecule VIII denaturing to form single stranded DNA molecules IX and X. Single stranded DNA molecule IX comprises a tail region 12 that is complementary to the tail region 10, and therefore complementary to the probe sequence 8. As illustrated in Fig. 1G, the probe 5 will therefore bind to the tail region 12 of molecule IX. During the elongation step, the 5'-3'exonuclease activity of a Tag polymerase will cleave the dual labelled probe, releasing the quencher label 7 into solution and allowing the fluorescent label to emit a detectable fluorescent signal. Fig. 1H shows a newly synthesised DNA molecule incorporating the fluorescent label 6.

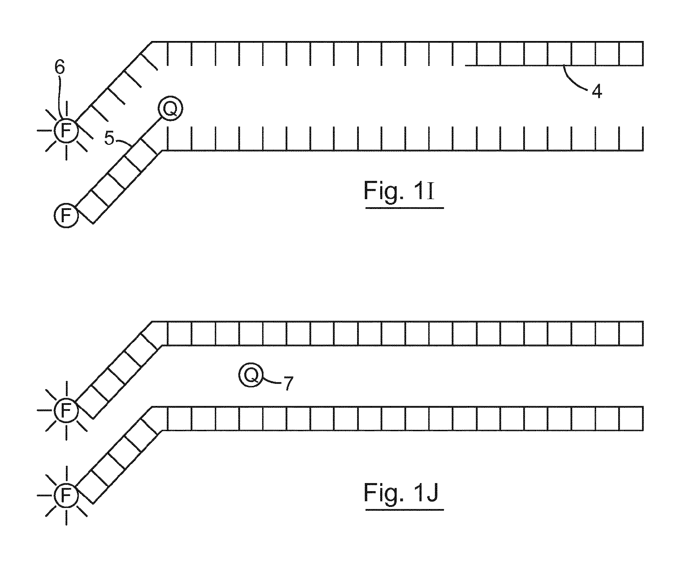

Referring to Fig. 1I, a fourth round of PCR is initiated with a denaturation step and the DNA molecule of Fig. 1H denaturing to form two single stranded DNA molecules, one comprising a tail region 12 that is complementary to the tail region 10, and therefore complementary to the probe sequence 8. The probe 5 therefore binds to the tail region and during the elongation step, the 5'-3'exonuclease activity of a Tag polymerase will cleave the dual labelled probe, again releasing the quencher label 7 into solution and allowing the fluorescent label to emit a detectable fluorescent signal. Fig. 1J shows a newly synthesised DNA molecules incorporating the fluorescent label 6 and providing continued amplification of signal and no loss of data/signal quality.

This embodiment of the invention may be employed to detect the presence of a specific target DNA molecule within a sample, by designing primer and probes that are specific for PCR amplification of the target sequence. In the next embodiment, the method of the invention may be employed to detect a specific allele of the target DNA molecule, for example an allele comprising a single nucleotide polymorphism, in which the reaction mixture comprises a forward PCR primer specific for a first allele of the target DNA and having a first tail, and a second forward PCR primer specific for a second allele of the target DNA and having a second tail, and two dual labelled probes, one having a probe sequence identical to the first tail and the other having a probe sequence identical to the second tail.

Referring to Figs 2A to 2H, there is illustrated a further embodiment of the invention, specifically a method of genotyping a sample of DNA for the presence of a first or second allele of a gene within the DNA by means of PCR amplification, in which parts identified with reference to Figs 1A to 1H are assigned the same reference numerals.

Referring initially to Fig. 2A, there is illustrated a first allele of a target DNA molecule I incorporating a single polynucleotide polymorphism (SNP) X, a first forward PCR primer 20A specific for the first allele of the target DNA molecule and having a first sequence 30A complementary to a sequence at the 5' end of the first strand of the target DNA molecule I, a second forward PCR primer 20B specific for the second allele of the target DNA molecule and having a first sequence 30B complementary to a sequence at the 5' end of the first strand of the target DNA molecule I, a common reverse primer 40 having a sequence complementary to a sequence at the 5' end of the second strand of the target DNA molecule I, a first probe 50A having a first fluorescent label 60A, a quencher 70, and a first probe sequence 80A, and a second probe 50B having a second fluorescent label 60B, a quencher 70, and a second probe sequence 80B. The first forward PCR primer 20A additionally comprises a tail sequence 100A that is identical to the first probe sequence 80A. The second forward PCR primer 20B additionally comprises a tail sequence 100B that is identical to the second probe sequence 80B. The figures do not illustrate the other components required to perform PCR, which include Taq DNA polymerase, deoxy nucleotide triphosphates dNTP's, and reaction buffer, the details of which will be known to a person skilled in the art.

Referring to Figs. 2B and 2C, a first round of PCR is initiated in the same way as described with reference to Figs. 1B and 1C. The first forward PCR primer 20A which is specific for the first allele of the target DNA molecule anneals to the first strand II and the common reverse primer anneals to the second strand III, and following elongation two new double stranded DNA molecules IV and V are formed, with molecule IV including the tail sequence 100A (Fig. 2C).

Referring to Fig. 2D, a second round of PCR is initiated with a denaturation step and the DNA molecule IV denaturing to form single stranded DNA molecules VI and VII. The first forward PCR primer 20A binds to the 5' end of strand VI, and the common reverse PCR primer 40 binds to the 5' end of the second strand VI, in an annealing step. As the probe sequence 80A of the probe 50A is identical with the tail sequence 100A of the primer 20A, it will not bind to the tail. Referring to Fig. 2E, following the elongation step, two new DNA molecules VIII and IX are synthesised, molecule VIII comprising the tail sequence 100A in a single stranded forms, and molecule IX comprising a double stranded tail region, with the tail sequence 100A having a complementary tail region 120A.

Referring to Fig. 2F, a third round of PCR is initiated with a denaturation step and the DNA molecule IX denaturing to form single stranded DNA molecules X and XI. Single stranded DNA molecule XI comprises a tail region 120A that is complementary to the tail region 100A of probe 50A, and therefore complementary to the probe sequence 80A. As illustrated in Fig. 2G, the probe 50A will therefore bind to the tail region 120A of molecule XI. During the elongation step, the 5'-3'exonuclease activity of a Tag polymerase will cleave the dual labelled probe, releasing the quencher label into solution and allowing the fluorescent label to emit a detectable fluorescent signal.

### Experimental examples

### First example

### Experimental description:

In the following example, samples that were previously tested and assigned SNP genotypes using KASP chemistry were selected to be re tested with the GASP chemistry. From these samples and KASP results, we were able to designate parentage trios (one male, one female, one offspring). We have selected 80 female parents, 80 male parents and 220 offspring. These samples had already been prepared for the KASP chemistry and the same samples were then retyped using GASP chemistry. In order to assess the success of the GASP chemistry in using one call, we compared both the first call and a typical re-cycling program identical to KASPs recommended re-cycling procedure. The method of the invention was performed according to the PCR cycling conditions shown in Table 1 and employing the procedure described in detail (in the "detailed description of the invention" section, referring specifically to figures 2A-2H). The template DNA for each sample was run against a panel of SNPs (65 in total, listed in Table 2a, 2b, and 2c.), each test for the presence of an allele consisted of particulars as described in paragraph 1, page 4. These primers, probes and template DNA were incubated together in a PCR mastermix (consisting of all the components necessary to perform PCR, with the exception of template, primers, and probes). Following on from this the PCR protocol was initiated in line with the cycling conditions as described in Table 1.

**Table 1: Specific PCR cycling conditions for GASP chemistry**

| Cycling Conditions | | |
|---|---|---|
| 95C | 5 minutes | 1 cycle |
| 95C | 10 seconds | 20 cycles |
| 60C | 60 seconds | |
| 95C | 10 seconds | 30 cycles |
| 66C | 60 seconds | |

**Table 2a. List of SNPs and the forward primers for the 1^{st} allele**

| **SNP Name** | **Forward Primer 1** |
|---|---|
| BASS113_B6A_F03_685 | GAAGGTGACCAAGTTCATGCTCAGTTACAACCCAGTACCTGTGTTAA |
| ESTNV_25635_721 | GAAGGTGACCAAGTTCATGCTGTGAAGTGGACTGCATGTCCGA |
| ESTNV_26827_412 | GAAGGTGACCAAGTTCATGCTGGATAATGCATAGTGCAATATTAGTGTATTA |
| ESTNV_29541_288 | GAAGGTGACCAAGTTCATGCTAAATATGGAGATATAATGATTCTATGCATTCTT |
| ESTNV_33681_253 | GAAGGTGACCAAGTTCATGCTAAGTCTATACTAGTCCTAGCACTCG |
| ESTNV_35432_2607 | GAAGGTGACCAAGTTCATGCTGTGTATGTGGAACATCCTCAGAGA |
| ESTNV_35456_667 | GAAGGTGACCAAGTTCATGCTAGGTGAGGGCCCTAGCTGG |
| ESTNV_35495_1382 | GAAGGTGACCAAGTTCATGCTAATAAAAGGCCTGCAATGCCTGAGA |
| ESTNV_36134_1190 | GAAGGTGACCAAGTTCATGCTCACTTCCTTAGTAATGACAATTCAAACT |
| ESTNV_36246_1265 | GAAGGTGACCAAGTTCATGCTATGGACAGTTGTGGTGCGCTTC |
| ESTNV_36618_208 | GAAGGTGACCAAGTTCATGCTCAGCAATGGACGCTTCTGGCT |
| ESTNV_36744_1266 | GAAGGTGACCAAGTTCATGCTAATATACTTCCGAAATAAATACTTTGTTTGATA |
| ESTNV_37607_394 | GAAGGTGACCAAGTTCATGCTCCCAGAGTAACTGTCAGGCTTC |
| ESTV_13419_141 | GAAGGTGACCAAGTTCATGCTGAGGTACCCCGTACCTGTTATTTTA |
| ESTV_15043_1033 | GAAGGTGACCAAGTTCATGCTGCGCACACCCATTTGTGTTGA |
| ESTV_15360_434 | GAAGGTGACCAAGTTCATGCTGTAGATAGGAAAGCTTCAGAGGAC |
| ESTV_15784_1161 | GAAGGTGACCAAGTTCATGCTCAGTGCCTTCCTGAAGAACGA |
| ESTV_16604_550 | GAAGGTGACCAAGTTCATGCTAGTTCATGTTACTTTGGAGTTCTCTGT |
| ESTV_16974_481 | GAAGGTGACCAAGTTCATGCTGTCAGAACACCTTGTCCACAGAT |
| ESTV_17239_1531 | GAAGGTGACCAAGTTCATGCTCAGAAGATCGTCGCTCCT |
| ESTV_17790_535 | GAAGGTGACCAAGTTCATGCTGGCAGAGGTGGACTGCCCT |
| ESTV_21226_650 | GAAGGTGACCAAGTTCATGCTTTGAGATCTGTGTCTGGAATCCT |
| GCR_cBin10025_Ctg1_195 | GAAGGTGACCAAGTTCATGCTGAAGAGTAGCATGTTTTCTTTGACCA |
| GCR_cBin1090_Ctg1_228 | GAAGGTGACCAAGTTCATGCTCGCTACACAACCTTGCTGAGG |
| GCR_cBin11286_Ctg1_158 | GAAGGTGACCAAGTTCATGCTGTACTGTAAGTAGGGTTAAAATAGATTTC |
| GCR_cBin12647_Ctg1_80 | GAAGGTGACCAAGTTCATGCTCCACCGGCTTAGATTCTTAAGTG |
| GCR_cBin12730_Ctg1_258 | GAAGGTGACCAAGTTCATGCTCAATGAAAGGCCAGGGAGAAATG |
| GCR_cBin13760_Ctg1_383 | GAAGGTGACCAAGTTCATGCTGGGATGGGAAGAGTGTTGGGAT |
| GCR_cBin14896_Ctg1_214 | GAAGGTGACCAAGTTCATGCTAACTGTATGGCGGTGTCTATAGG |
| GCR_cBin14940_Ctg1_245 | GAAGGTGACCAAGTTCATGCTGCACCACAAACACATCCTCCTCA |
| GCR_cBin1541_Ctg1_424 | GAAGGTGACCAAGTTCATGCTAGACAACACTGATGCGTTGTTTTGTTA |
| GCR_cBin1614_Ctg1_629 | GAAGGTGACCAAGTTCATGCTCCAGATTGCTCTTTGACTTGCGC |
| GCR_cBin16339_Ctg1_134 | GAAGGTGACCAAGTTCATGCTCAAACAGAGGCTCCTTCTCTCT |
| GCR_cBin17424_Ctg1_108 | GAAGGTGACCAAGTTCATGCTGGTAAAAGTAGATTACCCTCTCGGT |
| GCR_cBin2094_Ctg1_64 | GAAGGTGACCAAGTTCATGCTCAGAGGTCAGTACTGCTACCATA |
| GCR_cBin21603_Ctg1_118 | GAAGGTGACCAAGTTCATGCTACTGTTACATCATGTTGCTTTTGCCA |
| GCR_cBin22562_Ctg1_161 | GAAGGTGACCAAGTTCATGCTAGCGAACGAGATCCAAATTGCTCA |
| GCR_cBin22866_Ctg1_125 | GAAGGTGACCAAGTTCATGCTCCTCTGGATTAATGCTGTTTTACATCAT |
| GCR_cBin23367_Ctg1_256 | GAAGGTGACCAAGTTCATGCTTCAAGTTCTCATTTATTAGACGCCTC |
| GCR_cBin2400_Ctg1_488 | GAAGGTGACCAAGTTCATGCTAGTAGCAGCAATATTTGAGACATTCTG |
| GCR_cBin2433_Ctg1_97 | GAAGGTGACCAAGTTCATGCTCCAACTAGGAAACACTGAGGACG |
| GCR_cBin25838_Ctg1_135 | GAAGGTGACCAAGTTCATGCTAATTTCATTACTATAACTGCAGAACTGTTG |
| GCR_cBin26088_Ctg1_476 | GAAGGTGACCAAGTTCATGCTACAACTGGAGGGGCATCACACT |
| GCR_cBin27692_Ctg1_97 | GAAGGTGACCAAGTTCATGCTGAAGTGGCTGTGTGGTCCAAGT |
| GCR_cBin283_Ctg1_120 | GAAGGTGACCAAGTTCATGCTCAGTCAACAGCTGCCTGTGTGT |
| GCR_cBin28586_Ctg1_147 | GAAGGTGACCAAGTTCATGCTCGAAACGCGGCCGTCTCG |
| GCR_cBin28945_Ctg1_147 | GAAGGTGACCAAGTTCATGCTACATATTTACCCTCTTTAGGGTGCC |
| GCR_cBin29936_Ctg1_299 | GAAGGTGACCAAGTTCATGCTAGCCATTATTTCTCAAGAGCTGGGT |
| GCR_cBin3221_Ctg1_205 | GAAGGTGACCAAGTTCATGCTCATTTCGTTCAGCTAACGTTAGTAGTAT |
| GCR_cBin32932_Ctg1_86 | GAAGGTGACCAAGTTCATGCTCCTTAGCACAGCGTCCGCCA |
| GCR_cBin38765_Ctg1_214 | GAAGGTGACCAAGTTCATGCTCAGAACATTACCTCAGATAGAGTCG |
| GCR_cBin38782_Ctg1_147 | GAAGGTGACCAAGTTCATGCTCTTCCTGGTCTGCTTACCCG |
| GCR_cBin39462_Ctg1_188 | GAAGGTGACCAAGTTCATGCTGGGTTCTGATTGGTTGGTTGTGATA |
| GCR_cBin42907_Ctg1_150 | GAAGGTGACCAAGTTCATGCTAAATCTAAATGTTTTACTTGAATGGCATGC |
| GCR_cBin4585_Ctg1_148 | GAAGGTGACCAAGTTCATGCTGGTGTACCCCCAACGCTACTTT |
| GCR_cBin4710_Ctg1_83 | GAAGGTGACCAAGTTCATGCTAACCTGCCATATGCTTTTCACTCT |
| GCR_cBin49508_Ctg1_53 | GAAGGTGACCAAGTTCATGCTATATCGAAAACTACATTGATCCGCAAC |
| GCR_cBin5763_Ctg1_203 | GAAGGTGACCAAGTTCATGCTCCACAACGACAGACTCCATATTTTAT |
| GCR_cBin6010_Ctg1_524 | GAAGGTGACCAAGTTCATGCTGTGTGCCTCAGATTTGAGCATGATA |
| GCR_cBin628_Ctg1_209 | GAAGGTGACCAAGTTCATGCTCCGGTCATACCACTACAGTGACA |
| GCR_cBin6335_Ctg1_82 | GAAGGTGACCAAGTTCATGCTACCTCCATAAACAGGTGACTGTTTAA |
| GCR_cBin6366_Ctg1_223 | GAAGGTGACCAAGTTCATGCTCCTACCCCTTCTGTATCTCCTA |
| GCR_cBin8358_Ctg1_66 | GAAGGTGACCAAGTTCATGCTAAGCTCTGACAGCTTCTGATTCTCA |
| GCR_cBin8920_Ctg1_206 | GAAGGTGACCAAGTTCATGCTAATAGGGTGCCATTTAGGACACGAA |
| GCR_hBin30557_Ctg1_178 | GAAGGTGACCAAGTTCATGCTCTGTAGAACTGAGGTCTTACTTAGT |

**Table 2b. List of SNPs and the forward primers for the 2^{nd} allele**

| **SNP Name** | **Forward Primer 2** |
|---|---|
| BASS113_B6A_F03_685 | GAAGGTCGGAGTCAACGGATTAGTTACAACCCAGTACCTGTGTTAG |
| ESTNV_25635_721 | GAAGGTCGGAGTCAACGGATTGAAGTGGACTGCATGTCCGG |
| ESTNV_26827_412 | GAAGGTCGGAGTCAACGGATTGATAATGCATAGTGCAATATTAGTGTATTG |
| ESTNV_29541_288 | GAAGGTCGGAGTCAACGGATTATGGAGATATAATGATTCTATGCATTCTG |
| ESTNV_33681_253 | GAAGGTCGGAGTCAACGGATTAAGTCTATACTAGTCCTAGCACTCC |
| ESTNV_35432_2607 | GAAGGTCGGAGTCAACGGATTGTATGTGGAACATCCTCAGAGG |
| ESTNV_35456_667 | GAAGGTCGGAGTCAACGGATTCAGGTGAGGGCCCTAGCTGT |
| ESTNV_35495_1382 | GAAGGTCGGAGTCAACGGATTAAAAGGCCTGCAATGCCTGAGG |
| ESTNV_36134_1190 | GAAGGTCGGAGTCAACGGATTCACTTCCTTAGTAATGACAATTCAAACG |
| ESTNV_36246_1265 | GAAGGTCGGAGTCAACGGATTATGGACAGTTGTGGTGCGCTTG |
| ESTNV_36618_208 | GAAGGTCGGAGTCAACGGATTCAGCAATGGACGCTTCTGGCC |
| ESTNV_36744_1266 | GAAGGTCGGAGTCAACGGATTAATATACTTCCGAAATAAATACTTTGTTTGATG |
| ESTNV_37607_394 | GAAGGTCGGAGTCAACGGATTCCCAGAGTAACTGTCAGGCTTG |
| ESTV_13419_141 | GAAGGTCGGAGTCAACGGATTAGGTACCCCGTACCTGTTATTTTC |
| ESTV_15043_1033 | GAAGGTCGGAGTCAACGGATTCTGCGCACACCCATTTGTGTTGT |
| ESTV_15360_434 | GAAGGTCGGAGTCAACGGATTGTAGATAGGAAAGCTTCAGAGGAT |
| ESTV_15784_1161 | GAAGGTCGGAGTCAACGGATTCAGTGCCTTCCTGAAGAACGG |
| ESTV_16604_550 | GAAGGTCGGAGTCAACGGATTGTTCATGTTACTTTGGAGTTCTCTGG |
| ESTV_16974_481 | GAAGGTCGGAGTCAACGGATTGTCAGAACACCTTGTCCACAGAC |
| ESTV_17239_1531 | GAAGGTCGGAGTCAACGGATTGCTCAGAAGATCGTCGCTCCC |
| ESTV_17790_535 | GAAGGTCGGAGTCAACGGATTGGCAGAGGTGGACTGCCCA |
| ESTV_21226_650 | GAAGGTCGGAGTCAACGGATTTGAGATCTGTGTCTGGAATCCC |
| GCR_cBin10025_Ctg1_195 | GAAGGTCGGAGTCAACGGATTGAAGAGTAGCATGTTTTCTTTGACCC |
| GCR_cBin1090_Ctg1_228 | GAAGGTCGGAGTCAACGGATTCCGCTACACAACCTTGCTGAGA |
| GCR_cBin11286_Ctg1_158 | GAAGGTCGGAGTCAACGGATTGTACTGTAAGTAGGGTTAAAATAGATTTG |
| GCR_cBin12647_Ctg1_80 | GAAGGTCGGAGTCAACGGATTCCCACCGGCTTAGATTCTTAAGTA |
| GCR_cBin12730_Ctg1_258 | GAAGGTCGGAGTCAACGGATTGTCAATGAAAGGCCAGGGAGAAATT |
| GCR_cBin13760_Ctg1_383 | GAAGGTCGGAGTCAACGGATTGGATGGGAAGAGTGTTGGGAC |
| GCR_cBin14896_Ctg1_214 | GAAGGTCGGAGTCAACGGATTAACTGTATGGCGGTGTCTATAGC |
| GCR_cBin14940_Ctg1_245 | GAAGGTCGGAGTCAACGGATTCACCACAAACACATCCTCCTCC |
| GCR_cBin1541_Ctg1_424 | GAAGGTCGGAGTCAACGGATTGACAACACTGATGCGTTGTTTTGTTG |
| GCR_cBin1614_Ctg1_629 | GAAGGTCGGAGTCAACGGATTCCAGATTGCTCTTTGACTTGCGT |
| GCR_cBin16339_Ctg1_134 | GAAGGTCGGAGTCAACGGATTCAAACAGAGGCTCCTTCTCTCC |
| GCR_cBin17424_Ctg1_108 | GAAGGTCGGAGTCAACGGATTGTAAAAGTAGATTACCCTCTCGGC |
| GCR_cBin2094_Ctg1_64 | GAAGGTCGGAGTCAACGGATTCAGAGGTCAGTACTGCTACCATC |
| GCR_cBin21603_Ctg1_118 | GAAGGTCGGAGTCAACGGATTCTGTTACATCATGTTGCTTTTGCCG |
| GCR_cBin22562_Ctg1_161 | GAAGGTCGGAGTCAACGGATTGCGAACGAGATCCAAATTGCTCG |
| GCR_cBin22866_Ctg1_125 | GAAGGTCGGAGTCAACGGATTCTCTGGATTAATGCTGTTTTACATCAC |
| GCR_cBin23367_Ctg1_256 | GAAGGTCGGAGTCAACGGATTCAAGTTCTCATTTATTAGACGCCTG |
| GCR_cBin2400_Ctg1_488 | GAAGGTCGGAGTCAACGGATTTAGTAGCAGCAATATTTGAGACATTCTA |
| GCR_cBin2433_Ctg1_97 | GAAGGTCGGAGTCAACGGATTACCAACTAGGAAACACTGAGGACA |
| GCR_cBin25838_Ctg1_135 | GAAGGTCGGAGTCAACGGATTGAATTTCATTACTATAACTGCAGAACTGTTA |
| GCR_cBin26088_Ctg1_476 | GAAGGTCGGAGTCAACGGATTCAACTGGAGGGGCATCACACG |
| GCR_cBin27692_Ctg1_97 | GAAGGTCGGAGTCAACGGATTAAGTGGCTGTGTGGTCCAAGC |
| GCR_cBin283_Ctg1_120 | GAAGGTCGGAGTCAACGGATTCAGTCAACAGCTGCCTGTGTGA |
| GCR_cBin28586_Ctg1_147 | GAAGGTCGGAGTCAACGGATTGCGAAACGCGGCCGTCTCA |
| GCR_cBin28945_Ctg1_147 | GAAGGTCGGAGTCAACGGATTGACATATTTACCCTCTTTAGGGTGCT |
| GCR_cBin29936_Ctg1_299 | GAAGGTCGGAGTCAACGGATTCCATTATTTCTCAAGAGCTGGGC |
| GCR_cBin3221_Ctg1_205 | GAAGGTCGGAGTCAACGGATTTCGTTCAGCTAACGTTAGTAGTAG |
| GCR_cBin32932_Ctg1_86 | GAAGGTCGGAGTCAACGGATTCTTAGCACAGCGTCCGCCG |
| GCR_cBin38765_Ctg1_214 | GAAGGTCGGAGTCAACGGATTGCAGAACATTACCTCAGATAGAGTCA |
| GCR_cBin38782_Ctg1_147 | GAAGGTCGGAGTCAACGGATTCTCTTCCTGGTCTGCTTACCCA |
| GCR_cBin39462_Ctg1_188 | GAAGGTCGGAGTCAACGGATTGGGTTCTGATTGGTTGGTTGTGATT |
| GCR_cBin42907_Ctg1_150 | GAAGGTCGGAGTCAACGGATTAAATCTAAATGTTTTACTTGAATGGCATGG |
| GCR_cBin4585_Ctg1_148 | GAAGGTCGGAGTCAACGGATTGTGTACCCCCAACGCTACTTC |
| GCR_cBin4710_Ctg1_83 | GAAGGTCGGAGTCAACGGATTAACCTGCCATATGCTTTTCACTCG |
| GCR_cBin49508_Ctg1_53 | GAAGGTCGGAGTCAACGGATTATATCGAAAACTACATTGATCCGCAAG |
| GCR_cBin5763_Ctg1_203 | GAAGGTCGGAGTCAACGGATTCCACAACGACAGACTCCATATTTTAC |
| GCR_cBin6010_Ctg1_524 | GAAGGTCGGAGTCAACGGATTGTGCCTCAGATTTGAGCATGATC |
| GCR_cBin628_Ctg1_209 | GAAGGTCGGAGTCAACGGATTCGGTCATACCACTACAGTGACG |
| GCR_cBin6335_Ctg1_82 | GAAGGTCGGAGTCAACGGATTCCTCCATAAACAGGTGACTGTTTAC |
| GCR_cBin6366_Ctg1_223 | GAAGGTCGGAGTCAACGGATTCCTACCCCTTCTGTATCTCCTT |
| GCR_cBin8358_Ctg1_66 | GAAGGTCGGAGTCAACGGATTGCTCTGACAGCTTCTGATTCTCG |
| GCR_cBin8920_Ctg1_206 | GAAGGTCGGAGTCAACGGATTAGGGTGCCATTTAGGACACGAC |
| GCR_hBin30557_Ctg1_178 | GAAGGTCGGAGTCAACGGATTCTGTAGAACTGAGGTCTTACTTAGG |

**Table 2c. List of SNPs and the common reverse primers**

| **SNP Name** | **Common Primer** |
|---|---|
| BASS113_B6A_F03_685 | GACGCAATCTCAAGTCTCCCAAATATAAT |
| ESTNV_25635_721 | CGCATCAAACTGACGTGAAGCGTTT |
| ESTNV_26827_412 | TTCACAGAATGGATGCACAGAATTGGAAA |
| ESTNV_29541_288 | GTGCAAATGTACAGTTTCAGGACGGTA |
| ESTNV_33681_253 | GTGCATGTGACATGATTCGTTTTGATCTA |
| ESTNV_35432_2607 | GATGGGGTTGTTCTATGGCCAGAAT |
| ESTNV_35456_667 | GTGTACCAATGCCAGTCATTCTTATTGTA |
| ESTNV_35495_1382 | CTATTCCCACAAGCTTTCCTGTGGAT |
| ESTNV_36134_1190 | GACAGAGTACTTTTGATATCACAACTTGAA |
| ESTNV_36246_1265 | CCACAAGAGGGTGGTCTTTAGCTAT |
| ESTNV_36618_208 | TGTGAGGACCAGGAGGGCCAT |
| ESTNV_36744_1266 | GAGACAGGGCTATATCACTGCCATA |
| ESTNV_37607_394 | GGGAAGGTCTGTGCTTTAAGCTGTT |
| ESTV_13419_141 | TTGGCTAAAGTAAAAGCACTATTAGCACAA |
| ESTV_15043_1033 | GTCATGGGAAAAACCTTTTTCACTTCATAT |
| ESTV_15360_434 | ACCGTAAGACTGCCACCATCTGTTA |
| ESTV_15784_1161 | GACAGATTTGAATTGATAACACCGGTCTA |
| ESTV_16604_550 | CTTGCACTCCAAACTCAAGTTAACATCTT |
| ESTV_16974_481 | AAAGACTGACAGCAGGGGAAGACAA |
| ESTV_17239_1531 | ACTCATCGGCAGCAAGGATTCCTTT |
| ESTV_17790_535 | CATTCCGTGGAAGGTGACGGGTA |
| ESTV_21226_650 | AATTGTTGACCAACACTCGCACAAAGATT |
| GCR_cBin10025_Ctg1_195 | CGGTGTTGACTGTCCACTCTTTGTT |
| GCR_cBin1090_Ctg1_228 | TCATGTGCGGGTTCTGCTCATTCTA |
| GCR_cBin11286_Ctg1_158 | CAGCAATGCACAAGCTCAGTATCCAT |
| GCR_cBin12647_Ctg1_80 | CATCTTACTATGGAGAGAGGAACAGATTT |
| GCR_cBin12730_Ctg1_258 | AGTTCCCCCTCAGATATTAACTGUTTTTT |
| GCR_cBin13760_Ctg1_383 | CCAGATCTCCAGTAGTAGGCTGATT |
| GCR_cBin14896_Ctg1_214 | ACACTTTGTTGTACTTCTAACCAACATCTA |
| GCR_cBin14940_Ctg1_245 | CGTGGTGTTGGACGAGGCTGTA |
| GCR_cBin1541_Ctg1_424 | CTCCACTGTGCTCAGACCTCTATAT |
| GCR_cBin1614_Ctg1_629 | CCCCAAGACAGCTGTCCAAGGTA |
| GCR_cBin16339_Ctg1_134 | TCCAATGGGGTTTAGAAGCGAGCAA |
| GCR_cBin17424_Ctg1_108 | GGAGGTATCATGGACGATGTAAGGAT |
| GCR_cBin2094_Ctg1_64 | ATCTGTTTTTGCAAGAGAGACCTGTTCAA |
| GCR_cBin21603_Ctg1_118 | CAGTACAGTAAGTCACACTGGAGAAATAA |
| GCR_cBin22562_Ctg1_161 | GATAGATTACCCAAGGTGAGTTAAACAATT |
| GCR_cBin22866_Ctg1_125 | ATTCTATTGGATAAATACCTCTCCAACCAT |
| GCR_cBin23367_Ctg1_256 | GTCAGAGTTCATTCCTTCAGCACATTATT |
| GCR_cBin2400_Ctg1_488 | GGACACGCCACTATTGCTATTGTGTA |
| GCR_cBin2433_Ctg1_97 | CAGAGAGTTAAAGCTGCAAAATCTCACTT |
| GCR_cBin25838_Ctg1_135 | GGAATGGCGGATGGCAACAATATGTA |
| GCR_cBin26088_Ctg1_476 | CTTGCAAAATATCTTGCTGGGAATGTATAA |
| GCR_cBin27692_Ctg1_97 | CCGAAATGACAGATATGTTTAAGCTTGGAA |
| GCR_cBin283_Ctg1_120 | CCTACATACAGCCTGCTGCTACAAT |
| GCR_cBin28586_Ctg1_147 | GACTTCGTATCATACAGTATGCTTGTCAA |
| GCR_cBin28945_Ctg1_147 | GAGTTTATAACGCAGCAGAATATGACAATT |
| GCR_cBin29936_Ctg1_299 | CGGTGACTGATTGCATATTGTAGGAATTT |
| GCR_cBin3221_Ctg1_205 | TTGTTCAACACACAGTTGTTGGGAACTT |
| GCR_cBin32932_Ctg1_86 | GAGACTGCGGCAGGCAGATCAA |
| GCR_cBin38765_Ctg1_214 | CCTGCCCAGGTGGCTCCCAT |
| GCR_cBin38782_Ctg1_147 | CTTGGCTGTAAACCCATCCTACTCAA |
| GCR_cBin39462_Ctg1_188 | CCTGCTGGGCCTGAGAGAGAAA |
| GCR_cBin42907_Ctg1_150 | AAGGAGCATCACTTTATCAAATCCTCCTT |
| GCR_cBin4585_Ctg1_148 | GTCCCACTAAAACATTACATATGCAGAGTT |
| GCR_cBin4710_Ctg1_83 | TACACTGGCCAGTGTATCCTACATTATAT |
| GCR_cBin49508_Ctg1_53 | TAGACACAGTAGGGTTGGGGTGTAT |
| GCR_cBin5763_Ctg1_203 | TATGCTTTTGAGGCTTGTGTTGCAGAT |
| GCR_cBin6010_Ctg1_524 | GCCTCTCCAGATCACAGTCAGTTT |
| GCR_cBin628_Ctg1_209 | GGCACACGACACACAAACATGCTTT |
| GCR_cBin6335_Ctg1_82 | CCCGCTTCAACTAGCCTTAGCTTTT |
| GCR_cBin6366_Ctg1_223 | AGCTGGACAAGGCCAGAGCCTT |
| GCR_cBin8358_Ctg1_66 | GTATGGTTGTATTTTACATGTCGACTGGAT |
| GCR_cBin8920_Ctg1_206 | GAGATACTTTGTGGATACTGGCCCAA |
| GCR_hBin30557_Ctg1_178 | GCTAAAAGTTTTGTAAAAAGGGGCTTCAAT |

### Results

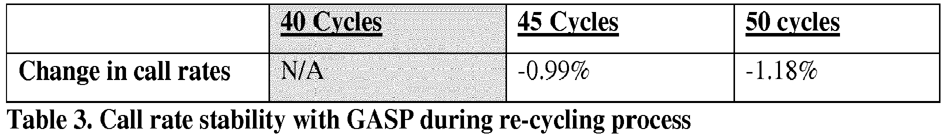

**Table 4. Concordance rates of KASP vs GASP - KASP scored independently and in its recommended fashion of multiple read analysis**

| | **GASP 1-cycle** | **GASP 3-cycle** |
|---|---|---|
| **SNP genotypes compared** | 16,494 | 16,770 |
| **% concordance** | 99.67% | 99.64% |

**Table 5. Mendelian inheritance - GASP SNP call % and parentage assignment concordance with KASP**

| | **No. of samples used** | **% SNPs typed** | **Assignment %** | **Assignment concordance with KASP** |
|---|---|---|---|---|
| **Female Parents** | 80 | 99.24 | N/A | N/A |
| **Male Parents** | 80 | 97.77 | N/A | N/A |
| **Offspring** | 220 | 98.51 | 100 | 100 |

### Conclusions

Samples previously tested with the KASP chemistry and analysed to assign parentage where re-tested using GASP single read and multiple read to see would the parentage assignment be equivalent to the previous KASP assignments.

In Table 3. it should be noted that unlike KASP none of the sample calls changed to a different call with over cycling, i.e. 100% concordance. Differences in call rates are down to rigidity of analyst calling. Unlike KASP where over cycling leads to call changes from 3 distinct groups into unclear scattergun plots or all samples merging into a single heterozygous called group, making a single read call difficult and inaccurate if sample quality and concentration is not consistent. It is a costly, if not sometimes impossible exercise, to insure high sample quality, as well as to normalise DNA concentration. Thus far these issues have not given cause for concern with the GASP chemistry exercise. SNP concordance rate with KASP is >99% in both the single read and multiple read analysis using the GASP chemistry (see Table 4.).

It can be concluded from these results that in real world, practical applications, the GASP chemistry single read is at least the equivalent of the KASP chemistry re cycling protocol.

### Second experimental example

### Experimental description

Fin clips were taken from a set of 3 specific salmon groups that were designated as female parents, male parents and offspring.

The aim of the experiment was to identify the family trio of male and female parent and their offspring using SNP genotyping and a panel of 65 markers (described in Table 2a, 2b, and 2c.) that were designed using a combination of previous in house experimental data and research that gave a statistically significant enough "fingerprint" to identify the parents of the offspring based on their genotypes and those of their parents. This would then be compared against a previous experiment where similar mating structures were observed and KASP chemistry was used.

### Experimental procedure (brief)

The fin clips were lysed in a proteinase K buffer overnight. These samples were then cleaned using a magnetic bead based DNA isolation kit (eg Agowa Mag mini DNA extraction kit).

After the samples were cleaned, the template DNA for each sample was run against a panel of SNPs (65 in total, listed in Table 2a, 2b, and 2c.), each test for the presence of an allele consisted of particulars as described in paragraph 1, page 4, (detailed more in depth in the Example of GASP chemistry in practice section, page 6)

. These primers (Table 2a, 2b, and 2c.), probes (as described in the "brief description of the invention", page 5) and template DNA were incubated together in a PCR mastermix (consisting of all the components necessary to perform PCR, with the exception of template, primers, and probes). Following on from this the PCR protocol was initiated in line with the cycling conditions as described in Table 1.

Once the PCR protocol had been run, and the alleles assigned, analysis of the SNP genotyping data obtained with the GASP chemistry was completed by our bioinformatics department. The results of the SNP genotyping were analysed using our in house data analysis software (there are several similar freeware types of this software online) that assigns parents to each offspring based on Mendelian inheritance of the specific SNPs that were genotyped.

We compare the results of this data to a similar project that had been previously run for the same purpose using solely KASP chemistry. While not a direct comparison it is an equivalent one.

### Results Summary

For the GASP chemistry, analysis of the results shows us that male parents were generally mated to ∼3 females, and females to ∼2 males

**Table 7. Results of parentage assignment with GASP chemistry**

| **Parentage test result** | **No. offspring** | **% of offspring** |
|---|---|---|
| Both parents assigned | 3006 | 91.6% |
| Female parent assigned | 94 | 2.9% |
| Male parent assigned | 63 | 1.9% |
| No parents assigned | 77 | 2.3% |
| QC fail | 43 | 1.3% |
| Total | 3283 | |

In comparison to a similar genotyping experiment carried out with KASP where male parents were generally mated to ∼2 females, and some females to multiple males

**Table 8. Results of parentage assignment with KASP chemistry**

| **Parentage test result** | **No. offspring** | **% of offspring** |
|---|---|---|
| Both parents assigned | 4192 | 90.7% |
| Female parent assigned | 312 | 6.8% |
| Male parent assigned | 2 | 0.0% |
| No parents assigned | 23 | 0.5% |
| QC fail | 92 | 2.0% |
| Total | 4621 | |

### Error rates

**Table 9. GASP chemistry error rates**

| **Description of error rate** | **% error (SNP calls)** |
|---|---|
| per locus Mendelian error rate | 0.016153846 |
| Highest per locus % error | 0.7 |
| per offspring Mendelian error rate | 0.017957635 |
| Highest per offspring % error | 3.1 |

**Table 10. KASP chemistry error rates**

| **Description of error rate** | **% error (SNP calls)** |
|---|---|
| per locus Mendelian error rate | 0.130137 |
| Highest per locus % error | 3.36 |
| per offspring Mendelian error rate | 0.160393812 |
| Highest per offspring % error | 3.3 |

### Conclusion

Samples that were never previously tested were tested with the GASP chemistry and then analysed to assign parents to offspring as trios. Calls were made using a single read and analysis also acknowledged any Mendelian errors that may have occurred. Errors such as this are generally due to miscalls of a SNP in either an offspring, leading to minor assignment issues, or in a parent, which can lead to large scale assignment issues as the error will affect all assignments of offspring to that parent.

In the GASP project there were 33 offspring samples with Mendelian errors out of 3163 samples (i.e. 1.04%), compared to the equivalent KASP project which had 395 offspring samples with Mendelian errors out of 4503 samples (i.e. 8.77%). Using our accepted threshold of allowing 2 errors per sample, we find that GASP had 3 unacceptable samples, 0.095% compared to KASP which had 8 unacceptable samples, 0.178%.

Results in Table 7 and 8 are divided into five categories. Form this data we see that both GASP and KASP had comparable assignments of both parents to their offspring with GASP slightly higher at 91.6% compared to KASPs 90.7%.

Again this data confirms our assertion that the GASP chemistry is at least comparable to the current KASP chemistry.

Scoring time was also analysed among the lab analysts, comparing the time necessary to score the KASP multiple reads (3 in this case) to the amount of time needed to score the GASP single read. On average there has been a reduction in scoring time of circa 75%, with times varying between analysts and data sets.

It can be concluded from these results that in real world, practical applications, the GASP chemistry single read is at least the equivalent of the KASP chemistry re cycling protocol and has numerous benefits including most importantly reduced scoring time and a reduction in error rates (i.e. miscalling of SNPs) with a >8 fold reduction in per locus Mendelian error rate and the same in per offspring Mendelian error rate (Table 9. vs. Table 10.).

The invention is not limited to the embodiments hereinbefore described which may be varied in construction and detail without departing from the spirit of the invention.

## Claims

1. A method for detecting PCR amplification of a target DNA molecule in a sample, which method employs:
- a forward PCR primer having a sequence that is complementary to the target double stranded nucleic acid and a tail sequence;
- a reverse PCR primer having a sequence that is complementary to the target double stranded nucleic acid; and
- a dual labelled probe containing an oligonucleotide sequence identical to the tail sequence of the forward PCR primer oligonucleotide, and a reporter label and a quencher-label separated by a nuclease susceptible cleavage site,
the method comprising the steps of:
- incubating the forward and reverse PCR primers and dual labelled probe together;
- performing at least two rounds of PCR to generate a double stranded nucleic acid comprising the tail region and a sequence complementary to the tail region; and
- initiating a further round of PCR in which the oligonucleotide probe binds to the sequence complementary to the tail region, whereby the oligonucleotide probe is displaced and cleaved resulting in a detectable signal from the reporter label.

2. A method as claimed in Claim 1 in which the reporter label is a fluorescent label.

3. A method for detecting the presence of a first allele of a target double stranded nucleic acid, which method comprises the step of carrying out the method of Claim 1 or 2, wherein the forward PCR primer is specific to the first allele, and wherein an increase in signal from the reporter label is indicative of the presence of the first allele of the gene in the sample.

4. A kit suitable for performing a method of detecting PCR amplification of a target double stranded nucleic acid in a sample, the kit comprising
- a forward PCR primer having a sequence that is complementary to the target double stranded nucleic acid and a tail sequence;
- a reverse PCR primer having a sequence that is complementary to the target double stranded nucleic acid; and
- a dual labelled probe containing an oligonucleotide sequence identical to the tail sequence of the forward PCR primer oligonucleotide, and a reporter label and a quencher-label separated by a nuclease susceptible cleavage site,

5. A method of genotyping a sample of DNA for the presence of a first or second allele of a gene within the DNA by means of PCR amplification, which method employs:
- a first forward PCR primer having a first allele-specific sequence and a first tail sequence;
- a second forward PCR primer having a second allele specific sequence and a second tail sequence different to the first tail sequence;
- a common reverse PCR primer;
- a first labelled probe comprising an oligonucleotide sequence identical to the tail sequence of the first forward PCR primer, and a first reporter label and a quencher-label separated by a nuclease susceptible cleavage site; and
- a second labelled probe containing an oligonucleotide sequence identical to the tail sequence of the second forward PCR primer, and a second reporter label and a quencher-label separated by a nuclease susceptible cleavage site, in which the second reporter label emits a signal that is different to that of the first reporter label,
the method comprising the steps of:
- incubating the forward and reverse PCR primers and probes together;
- initiating at least two rounds of PCR to generate a double stranded nucleic acid comprising the first or second tail region and a sequence complementary to the first or second tail region;
- initialling a further round of PCR in which one of the probes binds to the sequence complementary to the first or second tail region, whereby the oligonucleotide probe is displaced and cleaved resulting in a detectable signal from the reporter label; and
- correlating the detectable signal from the reporter label with the presence of the first or second allele of the gene in the sample of DNA.

6. A method as claimed in Claim 5 in which the reporter label is a fluorescent label.

7. A method as claimed in Claim 5 or 6 in which the first allele of the gene comprises a specific single nucleotide polymorphism and the second allele of the gene does not contain the single nucleotide polymorphism.

8. A kit suitable for genotyping a sample of DNA for the presence of a first or second allele of a gene within the DNA, the kit comprising:
- a first forward PCR primer having a first allele-specific sequence and a first tail sequence;
- a second forward PCR primer having a second allele specific sequence and a second tail sequence different to the first tail sequence;
- a common reverse PCR primer;
- a first labelled probe comprising an oligonucleotide sequence identical to the tail sequence of the first forward PCR primer, and a first fluorescent label and a quencher-label separated by a nuclease susceptible cleavage site; and
- a second labelled probe containing an oligonucleotide sequence identical to the tail sequence of the second forward PCR primer, and a second fluorescent label and a quencher-label separated by a nuclease susceptible cleavage site, in which the second fluorescent has excitation and emission properties that are different to those of the first fluorescent label.
